# EUROPEAN PATENT SPECIFICATION

(11) **EP 2 255 006 B1**
(45) Date of publication and mention of the grant of the patent: **18.09.2013**
(21) Application number: 09720086.9
(22) Date of filing: 12.03.2009
(51) Int. Cl.: C12N 15/82

(54) **PROCESS FOR PRODUCING TOMATO PLANTS WITH LONG-LIFE CHARACTERISTICS**
VERFAHREN ZUR HERSTELLUNG VON TOMATENPFLANZEN MIT LANGER LEBENSDAUER
PROCÉDÉ POUR PRODUIRE DES PLANTES DE TOMATE AYANT DES CARACTÉRISTIQUES DE LONGUE DURÉE DE VIE

(30) Priority: 13.03.2008 ES 200800836
(43) Date of publication of application: 01.12.2010
(73) Proprietor: Semillas Fito, S. A., 08019 Barcelona (ES)
(72) Inventor: JAHRMANN, Torben, E-08019 Barcelona (ES); GARCIA MAS, Jordi, E-08348 Cabrils, Barcelona (ES); PUJOL ABAJO, Marta, E-08348 Cabrils, Barcelona (ES); FITO CASTELLS, Eulalia, E-08019 Barcelona (ES)
(74) Representative: Pons Ariño, Angel
(86) International application number: PCT/EP2009/052912
(87) International publication number: WO 2009/112546

(56) References cited:
- WO-A-01/14561
- GIOVANNONI JAMES J ET AL: "Molecular genetic analysis of the ripening-inhibitor and non-ripening loci of tomato: A first step in genetic map-based cloning of fruit ripening genes" MOLECULAR AND GENERAL GENETICS, SPRINGER VERLAG, BERLIN, DE, vol. 248, no. 2, 1 January 1995 (1995-01-01), pages 195-206, XP002157273 ISSN: 0026-8925 cited in the application
- PALMIERI S ET AL: "INFLUENCE OF NR AND NOR GENES ON QUALITY AND STORAGE OF TOMATO FRUIT (LYCOPERSICON ESCULENTUM MILL.)" GENETICA AGRARIA, ISTITUTO SPERIMENTALE PER LA CEREALICOLTURA, ROME, IT, vol. 30, 1 January 1976 (1976-01-01), page 104/105, XP001134955 ISSN: 0016-6685
- MARTEL CATHERINE ET AL: "RIN and NOR function in acquisition of fruit ripening competency" PLANT BIOLOGY (ROCKVILLE), vol. 2007, 2007, page 220, XP009116955 & JOINT ANNUAL MEETING OF THE AMERICAN-FERN-SOCIETY/AMERICAN-SOCIETY-OF PLANT-BIOLOGISTS/AMERICAN-SOCI; CHICAGO, IL, USA; JULY 07 -11, 2007
- PALMIERI S ET AL: "Effects of the genes Nr and nor< on quality and storage life of tomatoes." ANNALI DELL'ISTITUTO SPERIMENTALE PER LA VALORIZZAZIONE TECNOLOGICA DEI PRODOTTI AGRICOLI 1975 ISTITUTO SPERIMENTALE PER L'ORTICULTURA DI SALERMO, SEZIONE OPERATIVA PERIFERICA DI MONTANASO, vol. 6, October 1975 (1975-10), XP009116957
- LOBO M ET AL: "INHERITANCE AND CHARACTERIZATION OF THE FRUIT RIPENING MUTATION IN CULTIVAR ALCOBACA TOMATO LYCOPERSICON-ESCULENTUM", JOURNAL OF THE AMERICAN SOCIETY FOR HORTICULTURAL SCIENCE, vol. 109, no. 5, 1984, pages 741-745, ISSN: 0003-1062

## Description

### Technical field

The present invention relates to the use os a mutation as a marker for identifying tomato plants, whose fruits have long-life characteristics.

### Background of the invention

The tomato (*Solanum lycopersicum L. or Lycopersicon esculentum*) is a plant from the Solanaceae family (*Solanaceae*) native to America and cultivated all over the world for its edible fruit.

Said fruit is a very coloured berry, with shades that typically change from yellowish to red, due to the presence of the licopene and carotene pigments. It has a lightly acidic flavour, a diameter of 1 to 2 cm in the wild species, and is much bigger in the cultivated varieties. It is produced and consumed all over the world both fresh and processed in different ways, either as a sauce, puree, juice, dehydrated or canned.

The tomato is a food having a low quantity of calories. In fact, 100 grams of tomato only have 18 kcal. Most of its weight is water and carbohydrates are the second important constituent. It contains simple sugars that confer it a light sweet flavour and some organic acids that provide the characteristic acidic flavour. The tomato is an important source of certain minerals as potassium and magnesium. From its vitamin content, we can highlight the B1, B2, B5 and the vitamin C.

In 2005 the worldwide production of tomatoes was up to 125 million tons, China being the main producer followed by the U.S. and Turkey.

The maturation of fleshy fruits like tomatoes represents the final stage of the fruit growth, by means of which the fruit obtains an aspect and a palatability that make it palatable for those organisms that will be in charge of disseminating the seeds.

Among the changes that happen during the process of maturation we can highlight: modifications of the cell wall texture and ultrastructure, conversion of starch into sugars, an increase of susceptibility to the attack by pathogenic agents after the harvest, alterations to the biosynthesis and accumulation of pigments, and high levels of aromas and volatile components.

The tomato attains its best organoleptical properties when it can mature in the plant with good sunshine and a high temperature. Nevertheless, when the tomato is mature, its consistency is reduced. This generally involves drawbacks to its distribution on the market in good condition.

The genetically modified tomatoes in order to retard their maturation, tend to last longer, but have less flavour and a more starchy and less attractive texture.

To overcome these drawbacks, a considerable number of investigation efforts have been addressed to study the biochemical bases of the tomato maturation process, the results of which have been published in numerous scientific works. A sample of this is the extensive reference list that is disclosed in the American patent US6762347.

Among prior art documents related to tomato maturation, we can mention the following:
- In the article Lincoln et al., Regulation of gene expression by ethylene during Lycopersicon esculentum (tomato) fruit development, PNAS USA, 1987, 84, 2793-2797, the role of the ethylene at molecular level on the tomato maturation is described, as the tomato belongs to a group of plants that present a high tax of formation of ethylene during their maturation, as well as high respiratory activity.
- In the article J. J. Giovannoni, Curr. Op. Plant Biol., 2007, 10, 1-7, the good perspectives offered by the mutant tomatoes for the maturation control comprehension are described.
- In the article Saladié et al., Proc. 5th Int. Postharvest Symp, Eds. F. Mencarelli and P. Tonutti, Acta Hort. 682, ISHS, 2005, it is described that the fruit cuticle of the tomato has an important role in the fruit's firmness and on the post-harvest attributes, as it undergoes a structural modification during the maturation, and that this could be an important factor of the fruit softening
- In the article Saladié et al., Plant Physiology, 2007, 144, 1012-1028, the metabolism contribution of the tomato peel to the fruit firmness from a tomato called DFD (from the English Delayed Fruit Deterioration) is described, said fruits undergoing a normal maturation, but remaining firm and not presenting any loss of integrity during considerable time periods once the grade of maturity has been reached.
- In the article Giovannoni et al., Mol. Gen. Genet., 1995, 1995, 248, 195-206, the characterization of two areas of the tomato genome that are responsible for an important inhibition of the maturation process is described: the inhibitor gene of the maturation, called *rin* (from the English *ripening inhibitor*) and the non-maturation gene called nor (from the English non ripening).
- In the article Vicente et al., J. Sci. Food Agric., 2007, 87, 1435-1448, the relationship between the cell wall metabolism and the fruit softening is described.
- In the article Mendes et al., Scientia agricola, 2003, 60(2), 269-275, it is described that the alc gene (alcobaça) has been demonstrated to be highly efficient for increasing the life of commercial tomatoes.
- In the patent application PCT WO-A-01 /14561 the sequence of the *nor* gene is described, as well as the creation of transgenic plants with the *nor* gene. It is also described that the new plants would present alterations in fruit characteristics and/or in their response against the ethylene, and that they would have one or more beneficial characteristics.

Despite the resorts intended to study the maturation process of the tomato from a molecular point of view, some unknown factors still remain to be solved.

Another approach for obtaining new vegetable varieties consists in crossing and selection processes that humanity has carried out for centuries in order to improve wild plants and obtain plants with improved organoleptical characteristics, having an improved yield and a lower sensitivity to attacks by pathogenic agents.

Said crossing and selection processes are generally long and laborious and require great experience from the horticulturist to select the exemplars that present the required phenotype, because of variability that occurs in the natural processes.

The necessity for having new procedures thus persists for the development of new tomato varieties, which have to be rapid, less laborious and less dependent on the horticulturist experience, and that will particularly allow the obtaining of tomatoes having long-life characteristics.

### Object of the invention

The object of the present invention is the use of the mutation present in the position 1109 of the nucleotidic sequence of the tomato nor gene as a marker for identifying tomato plants, whose fruits present long-life characteristics, for producing tomato plants with said characteristics, and for producing seeds that regenerate into tomato plants with long-life characteristics.

A process for identifying tomato plants, whose fruits present long-life characteristics is also part of the object of the invention.

A process for producing tomato plants, whose fruits present long-life characteristics is disclosed in the present invention.

A process for producing seeds that result in tomato plants, whose fruits present long-life characteristics is also disclosed in the invention.

### Figures

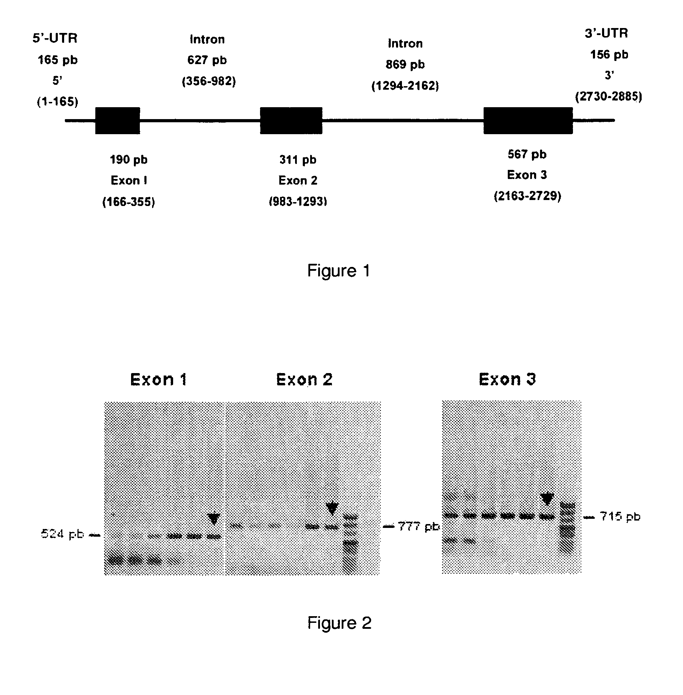

Figure 1. The nor gene has 3 exons. The exon 1 consists of 190 pairs of bases and is located between the positions 166 and 355 of the genomic nucleotidic sequence (registration number AY573803 in the GenBank of the National Center Biotechnology Information database). The exon 2 consists of 311 pairs of bases, and is located between the positions 983 and 1293 of the genomic nucleotidic sequence. The exon 3 consists of 567 pairs of bases and is present between the positions 2163 and 2729 of the genomic nucleotidic sequence. At the beginning of the nucleotidic sequence of the nor gene the 5'UTR of 165 pairs of bases is found between the positions 1 and 165. Between the exons 1 and 2 an intron of 627 pairs of bases is present, located between the positions 356 and 982. Between the exons 2 and 3 an intron of 869 pairs of bases is present, located between the positions 1294 and 2162. At the end of the nucleotidic sequence the 3'UTR of 156 pairs of bases is present placed between the positions 2730 and 2885.
Figure 2. Photography corresponding to the electrophoresis on agarose gel of the amplification products by PCR of the exons 1, 2 and 3 from the tomato nor gene obtained in the Example 1. The amplification product that includes the exon 1 has 524 pairs of bases, the one of the exon 2 has 777 pairs of bases, and the one of the exon 3 has 715 pairs of bases.

### Detailed description of the invention

The authors of the invention have discovered a mutation in the position 1109 of the nucleotidic sequence of the tomato nor gene that allows identifying tomato plants with fruits having some long-life characteristics, as well as to produce tomato plants with fruits having said characteristics and to produce seeds that regenerate into tomato plants, whose fruits present said long-life characteristics.

### ESL tomato plants

In the context of the invention, the tomato plants, whose fruits present long-life characteristics are those, whose fruits are preserved for a period of at least 3 months without the necessity of refrigeration, preferably for a period of at least 6 months.

Furthermore, fruits of said plants can maintain their organoleptical characteristics of juiciness and flavour during all the post-harvest period.

In this description the tomato plants, whose fruits present long-life characteristics are called ESL plants, from the English *Extended Shelf Life.*

### Use of mutation as a marker

The object of the present invention is to use the mutation consisting of the presence of adenine (A) instead of thymine (T) present in the position 1109 of the nucleotidic sequence of the tomato nor gene (SEQ_ID_NO:1), as a marker for identifying tomato plants, whose fruits are preserved during a period of at least 3 months without the necessity of refrigeration, to produce tomato plants with fruits having said characteristics, and to produce seeds that regenerate into tomato plants, whose fruits have long-life characteristics.

In the course of investigation works for the development of tomato plants with long-life characteristics, the authors of the invention have identified a type of native tomato with many varieties that presents said long-life characteristics.

The isolation works of the genomic DNA and the *nor* gene sequencing of different tomato varieties allow to determine that the tomatoes of the ESL type presented a mutation in the position 1109 of the nucleotidic sequence of the tomato *nor* gene. Tomatoes of the ESL type contain an adenine base (A) in said position, while further tomato varieties of the Cherry type, Pinton type, or tomato plants that incorporate the *rin* gene, contain a thymine base (T).

Said mutation responds to a polymorphism of a single nucleotide, that is usually known as SNP (from the *English Single Nucleotide Polymorphism*).

A SNP is a variation in the DNA sequence that occurs when a single nucleotide (A, T, C, or G) in the genome differs among members of a similar species.

It has been noted that to see a demonstration of the ESL phenotype, both alleles of the diploid plant must contain an adenine base (A) in the position 1109 of the *nor* gene.

The genomic DNA of the tomato nor gene (SEQ_ID_NO: 1) is recorded in the GenBank database of the NCBI (National Center for Biotechnology Information) with the registration number AY573803 and contains 2885 pairs of bases. The RNAm of the tomato nor gene (SEQ_ID_NO: 22) is recorded in said GenBank database with the registration number AY573802 and contains 1423 pairs of bases.

Comparing both sequences allows us to conclude that the *nor* gene presents three exons as shown in Figure 1. The exon 1 consists of 190 pairs of bases and is located between the positions 166 and 355 of the nucleotidic sequence. The exon 2 consists of 311 pairs of bases, and is between the positions 983 and 1293 of the nucleotidic sequence. The exon 3 consists of 567 pairs of bases and is between the positions 2163 and 2729 of the nucleotidic sequence. At the beginning of the nucleotidic sequence of the *nor* gene the 5'UTR of 165 pairs of bases is present between the positions 1 and 165. Between the exons 1 and 2 an intron of 627 pairs of bases located between the positions 356 and 982 is present. Between the exons 2 and 3 an intron of 869 pairs of bases located between the positions 1294 and 2162 is present. At the end of the nucleotidic sequence the 3'UTR of 156 pairs of bases located between the positions 2730 and 2885 is present.

The *nor* gene sequencing has been carried out according to processes well-known by the person skilled in the art, as for example by using the BigDye Terminator^{®} Kit from the Applied Biosystems company.

Said process includes the following steps: extraction of the genomic DNA from different varieties of tomato plants, amplification of the amplicon corresponding to the nor gene, and sequencing the same.

The extraction of the genomic DNA can be carried out for example according to the process disclosed in the article from Doyle et al., Focus, 1990, 12, 13-15.

Employing the polymerase chain reaction, which is usually known as PCR, it can be carried out the amplification of the genomic DNA.

To amplify the nor gene present in the extracted genomic DNA, the authors of the invention designed specific primers in order to amplify the corresponding nucleotidic sequences to exons 1, 2 and 3.

In Example 1 of this description the nucleotidic sequences of the primers that were employed for said amplification are included.

In the case of the exon 2, and due to the length of the same, two external primers and two internal primers were employed in two consecutive amplifications in order to achieve the appropriate amplification of the desired area.

Sequencing of the amplified genomic DNA can be carried out for example by employing the BigDye Terminator^{®} v 1.1 kit from the Applied Biosystems company.

The amplified sequences of the exons 1, 2 and 3 of different tomato varieties were aligned for identifying some possible polymorphisms. It has been proved that in exons 1 and 3 there was not a polymorphism between the tomatoes of the ESL type and the other tomato varieties of the cherry type and of Pinton type.

However, as mentioned previously, it has been proved that in the position 1109 of the nucleotidic sequence of the *nor* gene, that is present in the area corresponding to the exon 2, the tomatoes of ESL type presented a mutation in said position. The tomatoes of ESL type contained an adenine base (A) in said position, while other tomato varieties of the cherry type, Pinton type, or those incorporating the *rin* gene, contained a thymine base (T).

On carrying out the translation of the nucleotidic sequence into protein, it has been observed that said mutation caused an exchange of amino acid in the position 106 of the corresponding protein. The genotype T is coded by a valine aminoacid (non polar), and the genotype A by an aspartic acidic amino acid (polar).

### Process for identifying ESL tomato plants

The other aspect of the object of the present invention is a process for identifying tomato plants, whose fruits present long-life characteristics characterized in that it includes the step of identifying the presence of the adenine base (A) in both alleles in the position 1109 of the nucleotidic sequence of the tomato nor gene (SEQ_ID_NO: 1).

In the process of the invention, the identification of the tomato plants is carried out by means of well-known techniques by a person skilled in the art as for example the detection kit SNaPshot^{®}, a real time PCR employing sounds of the Taqman^{®} type from the Applied Biosystems company, or either Hyprobes^{®} type from the Roche^{®} company, or any further technology for detecting SNP polymorphisms.

In said process some specific primers are employed that are described in Example 2 and that allow to identify the tomato plants that contain the SNP in the position 1109 of the nucleotidic sequence of the tomato nor gene.

### Process for producing ESL tomato plants

A process for producing tomato plants, whose fruits present some long-life characteristics is also disclosed, comprising:
a) Providing a *Solanum lycopersicum* plant with alleles that contain an adenine base in the position 1109 of the tomato *nor* gene (SEQ_ID_NO: 1),
b) Crossing the provided plant in the step a) with culture material of *Solanum lycopersicum,* that presents a genotype AA or AT in the position 1109 of the tomato *nor* gene,
c) Collecting the resulting seeds from the crossing of step b),
d) Regenerating the seeds into plants, and
e) Identifying and selecting the plants by employing the marker of step a) that contain the genotype A in both alleles.

In one realization of the process of the invention, an ESL tomato plant (native plant, whose fruits presented long-life characteristics), which contained alleles that contained an adenine base in the position 1109 of the tomato nor gene, and that consequently presented a genotype AA, was crossed with another variety of tomato plant, for example of the Cherry or Pinton type, that presented a genotype TT in reference to said position.

From said crossing, seeds F1 were obtained that regenerated into a tomato plant. The extraction of the genomic DNA from a plant F1 was carried out as well as the identification of the base that was present in the position 1109 of the nor gene of said plant as described in the Example 2. It has been noted that said plant F1 was a hybrid that presented a genotype AT.

In an ulterior step, it is possible to collect seeds F2 from the plants obtained by self-fertilization, and to proceed to the extraction of the genomic DNA from various plants F2 and to identify the base that is in the position 1109 of the *nor* gene according to the process disclosed in Example 2.

With the use of the SNP or marker identified by the authors of the present invention it is possible to produce tomato plants, whose fruits combine the long-life characteristics with other interesting phenotypic characteristics from the commercial point of view, as for example size, shape, colour, or fruit texture.

For example, it is thus possible to produce tomatoes with long-life characteristics (ESL) that present characteristics of the tomato of the cherry type by employing the process of the invention. For this purpose, it is possible to cross a parental line of the ESL tomato that presents the genotype AA in the position 1109 of the *nor* gene, with a parental line of the cherry type that presents the genotype TT in said position. From this form an F1 that presents a genotype AT is obtained. On crossing the hybrid F1 with an ESL parental line, an F2 is obtained and the plants that present the genotype AA can be selected by employing the marker of the invention. By employing the backcross or backcrossing process, well-known by a person skilled in the art, and the marker of the invention to select the plant with the genotype AA, it is possible to obtain parental lines with long-life characteristics (ESL) and other commercially interesting characteristics as those of the cherry tomato.

### Process for producing seeds of ESL tomato plants

It is also disclosed a process for producing seeds that result in tomato plants whose fruits have some long-life characteristics that comprise the steps of:
a) providing a *Solanum lycopersicum* plant with alleles that contain an adenine base in the position 1109 of the tomato *nor* gene SEQ_ID_NO:1,
b) crossing the provided plant in the step a) with culture material of *Solanum lycopersicum,* that presents a genotype AA or AT in the position 1109 of the tomato *nor* gene,
c) collecting the seeds resulting from the crossing of step b), and
d) identifying and selecting the seeds by employing the marker of step a) that contain the genotype A in both alleles.

Surprisingly, it has been found that the fruits of tomato plants that contain an adenine base (A) in both alleles in the position 1109 of the *nor* gene present some long-life characteristics, so that they can be preserved during a period of at least 3 months, and generally of at least 6 months without the necessity of refrigeration, in order to reduce the storage and transport costs, and likewise the fruit keeps its organoleptical characteristics of juiciness and flavour throughout the post-harvest period.

### Process for producing long-life plants (ESL) by transgenesis.

Although the plant with the mutation in homocigosis has the more interesting characteristics, such as those described in the examples of the present invention, a plant can contain a mutation that presents the adenine base (A) in the position 1109 of the *nor* gene in homocigosis, heterocigosis or hemicigosis.

As above mentioned, the plant can be obtained by crossing, i.e. by employing pollen from the homocigotic plant for the polymorphism that presents an adenine base in the position 1109 of the *nor* gene for pollinating any other plant that does not contain said mutation in the nor gene sequences, by pollinating the gineceo of plants that contain the mutated sequence of the invention with another plant's pollen that does not contain this sequence, or can be obtained by genetic transformation mediated by biolistics, infection by *Agrobacterium,* or any other technique known by the person skilled in the art that allows the integration of any of the sequences of the invention into the plant's DNA, either nuclear, chloroplastic or mitocondrial.

It is also disclosed the use of:
a) SEQ ID NO: 1 that presents an adenine base (A) in the position 1109, instead of a thymine base (T),
b) a sequence homologous to the nucleotidic sequence of (a), that presents an adenine base (A) in the position homologous to the 1109, or
c) a genetic construction (thereinafter genetic construction of the invention) that includes the nucleotidic sequence according to (a), (b), or any of their combinations,
for producing long-life plants (ESL).

It can be expected that the identity/similarity grade of the sequences homologous to the collection in the sequence SEQ ID NO: 1 is, in different species inside the *Solanum* genus, and inside the different varieties and subspecies of *Solanum lycopersicum* L., at least of 80% or greater, and more preferably at least of 85%, 90, 95% or 99%. The correspondence between the nucleotitic sequence(s) of the adoptive sequence(s) and the sequence collected in SEQ ID NO: 1 can be determined by methods known from the technique. The methods for comparing the sequences are known in the state of the art, and include, without limitation, the BLASTP or BLASTN programme, and the FASTA programme (Altschul et al., J. Mol. Biol. 215, 403-410 (1999). Preferably, the differences (mutations) presented by the nucleotidic sequence homologous with the SEQ ID NO: 1 are present in non-coding regions, without affecting the product of the expression of said sequence.

The term "homology", as it is used in this description, thus relates to the likeness between two structures due to a common evolutionary ascendancy, and more specifically, to the likeness between two or more nucleotide or amino acid sequences. It can be considered that homologous sequences, with an identity of at least 80% with the nucleotidic sequence of the SEQ ID NO: 1, which presents an adenine base (A) in the position homologous to 1109, would have the same function.

The genetic construction of the step (c) of the invention, does not correspond to a gene expression system or vector comprising the SEQ ID NO: 1, that presents the adenine base (A) in the position 1109 of the *nor* gene instead of thymine (T), or the sequence homologous to the SEQ ID NO: 1 that presents an adenine base (A) in the position homologous to 1109, operatively linked to at least one promoter that conducts the transcription of said nucleotides sequence of, and with further necessary or appropriate sequences for the transcription and its adequate regulation in regard to time and location, for example, starting and ending signals, cutting sites of the polyadenylation signal, replication origin, transcriptional activators (*enhancers*), transcriptional silencers (*silencers*), etc.

A great number of these constructions, systems or vectors of expression can be achieved by conventional methods known by the persons skilled in the art and are part of the present invention.

A "vector" is a replicon, or an integrative vector, to which another polinucleotide segment is joined, in order to carry out the joined segment replication and/or expression.

A "replicon" is any genetic element that acts as an autonomous unit of polynucleotidic replication within a cell; which is able to replicate itself on its own.

An integrative vector is any genetic element that integrates into and is maintained stable in the genome of a cell.

"Control sequence" relates to sequences of polynucleonutides that are necessary to carry out the expression of the sequences to which they are linked. As it is used herein, the term "promoter" relates to an upper region of the DNA from the starting point of the transcription, and in particular, that it is able to start the transcription in a vegetal cell, whether the origin of the promoter is or is not a plant.

Joined in an "operative" way refers to a juxtaposition where the components described like this have a relation that allows them to work in an intentional way. A control sequence "joined in an operative way" to a sequence that is transcripted to the nucleotidic sequence of the invention is linked in such a manner that the expression of the coding sequence is achieved under conditions compatible with the control sequences.

A "coding sequence" or "encoding sequence " is a sequence of polynucleotides that is transcripted to mRNA and/or is translated to a polypeptide when it is under the control of appropriate regulated sequences.

The invention further discloses a process for producing tomato plants, whose fruits present some long-life characteristics that comprises:
a) transfection of the SEQ ID NO: 1 that presents an adenine base (A) in the position 1109, instead of a thymine base (T), a sequence homologous to the nucleotidic sequence SEQ ID NO: 1, that presents an adenine base (A) in the homologous position to 1109, or a genetic construction that comprises the sequence SEQ ID NO: 1 that presents the adenine base (A) in the position 1109 (or a sequence homologous to the nucleotidic sequence SEQ ID NO: 1, that presents an adenine base (A) in the position homologous to 1109), in a cell or a culture of host vegetal cells,
b) growing the cell or culture of host vegetal cells in adequate means until the regeneration of a complete plant.

"Transfection" or "transgenesis" are understood in this description as the process for transferring improper DNA to an organism, which thus becomes called "transgenic".

The term "transgenic" is used in the context of the present invention to describe plants wherein an improper DNA sequence, in a preferably stable form, has been incorporated and specifically the polynucleotides or genetic constructions of the invention.

Preferably, the host transfected cell can be taxonomically qualified as a member of the *Solanum* genus. More preferably, the transfected cell can be taxonomically qualified as a member of the *Solanum lycopersicum* species.

A "host", "host cell" or "hosting cell" as it is employed in this description relates to an organism, cell or tissue, particularly to a vegetal cell, which is used as a target or vessel for the transfected elements (for example, polynucleotides or genetic constructions of the invention)

With "plant" in this description, we understand all the organisms that can be qualified within the *Viridiplantae* realm, including the green algae and the land plants (*Embryophyta*). The organisms of the *Solanum* genus pertain to the *Eukaryota* Super Realm, *Viridiplantae* Realm, Phylum *Streptophyta,* Subclass *Asteridae,* Order *Solanales, Solanaceae* Family. *Solanum lycopersicum* is the scientific name of the tomato plant.

The terms "polynucleotide" and "nucleic acid" are used herein in an interchangeable manner, referring to polymeric forms of nucleotides of any length, both ribonucleotides (RNA or ARN) as desoxiribonucleotides (DNA or ADN).

The terms "aminoacidic sequence", "peptide", "oligopeptide", "protein" and "polypeptide" are used herein in an interchangeable manner, and refer to a polymeric form of amino acids of any length, which can or cannot be, chemically or biochemically modified.

The cell transformed with a vector that includes the SEQ ID NO: 1, that presents the adenine base (A) in the position 1109 of the nor gene instead of thymine (T), can incorporate the sequence in one of the DNA of the cell; nuclear, mitocondrial and/or chloroplastic, or remain as a part of a vector that has its proper machinery for its auto-replication.

More preferably, the cell transformed with a vector that includes the SEQ ID NO: 1, which presents the adenine base (A) in the position 1109 instead of thymine (T), or a sequence homologous to the nucleotidic sequence SEQ ID NO: 1, that presents an adenine base (A) in the position homologous to the 1109, instead of a thymine base (T), can incorporate the sequence in one of the DNA of the cell; nuclear, mitocondrial and/or chloroplastic in a stable form. Methods for the incorporation of an exogenous nucleic acid in the genome of a vegetal cell in a stable form are known in the state of the art.

The transformed cells, if they are vegetal, can be submitted to a somatic organogenesis or embryogenesis programme by means of which, after the dedifferentiation of the same by an adequate combination of vegetal hormones and other compounds, it results in a complete plant that contains the genetic material of the original cell from which it has originated.

The following examples are given in order to provide a detailed explanation of concrete embodiments within the invention for the person skilled in the art.

### Preparatory example- Extraction of genomic DNA from tomato plants

The process used to extract the genomic DNA from tomato plants substantially matches up with the process described in Doyle et al., Focus, 1990, 12, 13-15.

The extraction of genomic DNA from tomato plants of the ESL type, the cherry type, the Pinton type, and from tomato plants that incorporated the *rin* gene in their genome was carried out.

A young leaf was placed in a 1.9 ml eppendorf tube, and this was immersed in liquid nitrogen. The leaves were then crushed, 500 µl of the buffer solution from Doyle extraction previously heated at 65° C was added, and this was gently agitated.

The sample was left in a water bath at 65° C for 30 minutes.

After this time period a mixture of chloroform and isoamylic alcohol in a ratio 24:1 volume/volume was rapidly added.

This was centrifuged at 11,000 rpm at room temperature for 10 minutes, and 200 µl of the aqueous phase was transferred to a new eppendorf tube.

350 µl of isopropyl alcohol was added at 4° C to the aqueous phase, and this was gently mixed until the formation of DNA threads.

This was centrifuged at 10,000 rpm at room temperature during 5 minutes, and the supernatant was eliminated with a pipette. In the eppendorfs bottom part a whitish precipitate was present that was the extracted DNA.

To such a precipitated 200 µl of Doyle wash buffer was added, this was centrifuged at 10.000 rpm at room temperature for 3 minutes, and the supernatant was eliminated. The obtained pellet was dried, and resuspended in 50 µl of water of HPLC type. This was left overnight at 4° C, and was subsequently kept at -20° C. Said suspension was used to achieve the sequencing of the nor gene exons from each one of the tested tomatoes.

The Doyle extraction buffer and the Doyle wash buffer are described in the article already mentioned by Doyle *et al..*

### Example 1.- Sequencing of tomato nor gene exons

The sequencing of the nor gene exons of different types of tomato was carried out by using the polymerase chain reaction (PCR) to amplify the exons of said gene, and its sequencing was then carried out, with a previous amplification and marking.

The sequencing of the genomic DNA of tomato plants of the ESL type, the cherry type, the Pinton type and tomato plants that incorporated the *rin* gene in its genome was carried out.

### 1. Amplification of the exons

To amplify each one of the three exons of the tomato nor gene, 0.5 l (equivalent to 20-100 ng) of genomic DNA obtained in the preparatory Example, was mixed with 15 µl of distilled water, 2.5 µl PCR of buffer x10conc (AmpliTaq 10x PCR buffer II, Applied Biosystems), 2.5 µl of 25 mM MgCl₂ solution (AmpliTaq MgCl₂ Solution, Applied Biosystems), 2 µl of 5 mM dNTPs, 1 µl of a solution of the specific primer F for each 10 µM exon, 1 µl of a solution of the specific primer R for each 10 µM exon, and 0.5 µl of Taq DNA Polymerase (Applied Biosystems).

The AB9700 thermocycler programme of Applied Biosystems was executed according to the following sequence: 1 minute at 94° C; 35 repetitions of the cycle of 30 seconds at 94° C, 30 seconds at 60° C, 90 seconds at 72° C, ending with a cycle of 5 minutes at 72° C.

Once the amplification finished, it was checked by an agarose gel electrophoresis according to methods well-known by the person skilled in the art.

Primers employed for the amplification of the three exons of the tomato *nor* gene are shown in Table I:

**TABLE I**

| Exon | Reference | Primer sequence | SEQ_ID_NO: |
|---|---|---|---|
| 1 | External primer F | TCATTTTCTCTCTTCCCAAAAA | 3 |
| | External primer R | CATGAAGATTAAAAAGGGGAAA | 4 |
| 2 | External primer F | TCCACGATAACGTGTAAAAAGTG | 5 |
| | External primer R | TTTTCATCAAAAATTTGAAAGTTGA | 6 |
| | External primer F | TTCGGAGAGCAAGAATGGTT | 7 |
| | External primer R | CAACCAAGTGGCTTGTTATTTG | 8 |
| 3 | External primer F | AACTCGAATAAATTCAATGTTTCAT | 9 |
| | External primer R | TCGATTGATTTTACAGGGCTAA | 10 |

In the case of the exon 2, that has 777 pairs of bases, two amplifications were carried out: firstly with both external primers, and secondly with the internal primers.

In Figure 2, the result of the agarose gel electrophoresis is shown for the exons 1, 2 and 3 of the tomato *nor* gene.

Before proceeding to the amplification and marking of the sequences of the exons to carry out their sequencing, a purification of the same can possibly be carried out by using the High Pure PCR Product Purification Kit^{®} kit from Roche Applied Science.

### 2.- Sequencing of exons

The sequencing of the three exons was carried out by using the BigDye Terminator^{®} v1.1 sequencing kit from the Applied Biosystems company.

The sequencing of each one of the genomic DNA that were obtained from different tomato types of the preparatory Example after being amplified by PCR was carried out with the primers described in the Table I.

4 µl of the PCR product from the three exons were placed as disclosed in the section 1 of the Example 1, 2 µl of the reactivate BigDye Terminator^{®} (Applied Biosystems), as well as 1 µl of a solution of the specific external primer for each 3.2 mM exon, and 3 µl of distilled water.

The following programme was executed in the thermocycler AB9700: 25 cycles of 10 seconds at 96° C, 5 seconds at 50°C, and 4 minutes at 60° C.

Following the provider instructions of the kit, this was precipitated with a mixture of ethanol, EDTA and sodium acetate.

The obtained precipitate was resuspended in an injection buffer from the Applied Biosystems Company, and the suspension was loaded in the automatic sequencer ABI PRISM 3130 from the Applied Biosystems Company, and the sequencing programme was executed.

The sequencing data was analyzed by means of using the software programme GeneMapper^{®} from the Applied Biosystems company.

It was noticed that the tomato of the ESL type contained in the position 1109 of the *nor* gene, specifically in the exon 2 of said gene, one thymine (T), while tomatoes of the cherry type, of the Pinton type, or tomatoes that included the *rin* gene in their genome had one adenine (A) in said position.

By carrying out the translation of the nucleotidic sequence into protein, it was observed that said mutation caused an amino acid exchange in the position 106 of the corresponding protein. The genotype T codes for a valine amino acid (not polar), and the genotype A codes for an aspartic acidic amino acid (polar).

### Example 2. Identification of tomatoes of the ESL type

The identification of tomatoes of the ESL type was achieved by using the PCR technique according to the process described as follows wherein some primers designed to be able to rapidly identify the base that is present in the position 1109 of the exon 2 of the tomato nor gene are employed.

Firstly the genomic DNA was extracted according to the process disclosed in the preparatory Example.

Then 0.5 µl of genomic DNA was mixed corresponding to 20-100 ng, 15 µl of water, 2.5 µl of PCR buffer x10conc (AmpliTaq 10x PCR buffer II, Applied Biosystems), 2.5 µl of 25 mM MgCl₂ solution (AmpliTaq MgCl₂ Solution, Applied Biosystems), 2 µl of 5 mM dNTPs, 1 µl of a solution of primer F (SEQ_ID_NO: 7) 10 µM, 1 µl of a solution of primer R (SEQ_ID_NO: 8) 10 µM, and 0.5 µl (2.5 u) of Taq DNA Polymerase (AmpliTaq^{®}- Applied Biosystems).

The amplification was carried out in a AB9700 thermocycler, according to the following sequence: 1 cycle of one minute at 94° C; 35 cycles of 30 seconds at 94°C, 30 seconds at 60° C, and 30 seconds at 72° C; ending with a cycle of 5 minutes at 72° C.

It has been checked that the amplification was correct by means of an electrophoresis on agarose gel according to methods well known by the person skilled in the art.

2.5 µl of the amplified product was transferred to a new tube and 1 µl of ExoSAP-IT from the Applied Biosystems Company was added. It was mixed and a centrifuge punch was applied.

The product was then incubated in a thermocycler for 15 minutes at 37° C, and for 15 minutes at 80° C.

To carry out the SNaPShot reaction, 1.5 µl of the obtained DNA, 1.25 µl of the reactivate Mix SNaPShot (Applied Biosystems), 0.5 µl of a solution of the internal primer (SEQ ID NO: 11) 2 µM, and 1.75 µl of HPLC water (Panreac) were mixed together.

The internal primer used in this step had the sequence 5'-TACCGGAACCGACAAGCCGG -3' (SEQ_ID_NO: 11).

The PCR was executed with the next programming of the thermocycler: 25 cycles of 10 seconds at 96° C, 5 seconds at 50° C, and 30 seconds at 60° C, and was finally maintained at 4° C.

1 µl of CIP (1u/µl) (calf intestinal phosphatase) was added, agitated and a centrifuge punch was applied.

This was incubated in a thermocycler for 1 hour at 37° C, 15 minutes at 75° C, and maintained at 4° C.

0.5 µl of the product obtained was then loaded in a sequencing plate together with 12 µl of HI-DI formamide, and 0.5 µl of the reactivate LIZ 500 size standard.

This was denatured for 5 minutes at 95° C, the plate was cooled with ice and a centrifuge punch was applied therein.

The AB3130 automatic sequencer (SNP, E5 matrix, FragmentAnalysis36_POP7_1 module) was loaded and the data was analyzed with the software programme GeneMapper^{®} from the Applied Biosystems company.

The Table II presents the results obtained by applying the process of the invention to different tomato lines:

**TABLE II**

| Tomato line | Genotype | Phenotype | Life period | Fruit color |
|---|---|---|---|---|
| 1 | TT | Cherry | 10 days | Red |
| 2 | AA | ESL | > 6 months | Orange |
| 3 | TT | Pinton | 10 days | Red |
| 4 | TT | Pinton | 10 days | Red |
| 5 | TT | Pinton | 10 days | Red |
| 6 | AT | Hybrid | 30 days | Red |
| 7 | TT | rin | 60 days | Yellow |
| 8 | TT | rin | 60 days | Yellow |
| 9 | TT | rin | 60 days | Yellow |
| 10 | TT | rin | 60 days | Yellow |
| 11 | AA | ESL | > 6 months | Orange |
| 12 | AA | ESL | > 6 months | Orange |
| 13 | AA | ESL | > 6 months | Orange |
| 14 | AA | ESL | > 6 months | Orange |
| 15 | AA | ESL | > 6 months | Orange |
| 16 | AA | ESL | > 6 months | Orange |
| 17 | AA | Hybrid F1 | > 6 months | Orange |
| 18 | AA | Hybrid F1 | > 6 months | Orange |
| 19 | AA | Hybrid F1 | > 6 months | Orange |

It can be noted that the tomatoes of ESL type, that have a genotype AA in the position 1109 of the *nor* gene, present a life period greater than 6 months, while the tomatoes that present a genotype TT do not present said characteristic.

Hybrids AT obtained from an ESL parental line with a genotype AA and a parental line with a genotype TT do not present the long-life characteristics either.

The hybrid lines 17 to 19 have been obtained from two parental lines that present the genotype AA.

### Example 3. Production of tomatoes of the ESL type

A tomato plant of the ESL type was taken that had the genotype AA in the position 1109 of the *nor* gene, and this was crossed with a tomato plant of the Cherry type that had the genotype TT in said position.

A first generation F1 was obtained that was submitted to the identification assay described in the Example 2, and it was noted that they presented an hybrid genotype AT.

The crossing of a tomato plant of the ESL type with the hybrid AT involves obtaining plants, and by using the marker of the invention, those plants that present long-life characteristics (ESL) with the genotype AA in the position 1109 of the *nor* gene are selected.

By using the backcross or backcrossing technique, well-known by the person skilled in the art, some ESL parental lines can be obtained that present characteristics of another tomato type as for example the cherry type.

## Claims

1. Use of the mutation consisting of the presence of adenine, A, instead of thymine, T, present
a. in the position 1109 of the nucleotidic sequence of the tomato *nor* gene SEQ ID NO: 1 or
b. in the corresponding position with regard to the position 1109 of SEQ ID NO: 1, of sequences having an identity of at least 80% with SEQ ID NO: 1,
as a marker for identifying tomato plants having the genotype AA, whose fruits, which present long-life characteristics, are preserved for a period of at least 3 months without the necessity of refrigeration.

2. The use according to claim 1, wherein additionally are selected the identified tomato plant having the genotype AA in the position 1109 as described in claim 1a or 1b.

3. The use according to claim 1, wherein additionally is selected at least a seed from the identified tomato plant having the genotype AA in the position 1109 as described in claim 1a or 1b.

4. The use according to any of claims 1 to 3, wherein the tomato plant is taxonomically qualified in the *Solanum lycopersicum* species.

## Patentansprüche

1. Verwendung der Mutation, die in der Gegenwart von Adenin, A, anstelle von Thymin, T, besteht und
a. in der Position 1109 der Nukleotidsequenz der SEQ ID Nr. 1 des Tomaten-*nor*-Gens oder
b. in der entsprechenden Position in Bezug auf die Position 1109 von SEQ ID Nr. 1 von Sequenzen, die eine Identität von mindestens 80% mit SEQ ID Nr. 1 haben, vorliegt,
als ein Marker zum Identifizieren von Tomatenpflanzen mit dem Genotyp AA, deren Früchte, die Langlebigkeitscharakteristika aufweisen, für einen Zeitraum von mindestens 3 Monaten ohne Erfordernis einer Kühlung aufbewahrt werden.

2. Verwendung nach Anspruch 1, wobei zusätzlich die identifizierte Tomatenpflanze mit dem Genotyp AA in der Position 1109, wie in Anspruch 1a oder 1 b beschrieben, ausgewählt wird.

3. Verwendung nach Anspruch 1, wobei zusätzlich mindestens ein Samen von der identifizierten Tomatenpflanze mit dem Genotyp AA in der Position 1109, wie in Anspruch 1 a oder 1 b beschrieben, ausgewählt wird.

4. Verwendung nach einem der Ansprüche 1 bis 3, wobei die Tomatenpflanze taxonomisch in die Spezies Solanum lycopersicum eingestuft ist.

## Revendications

1. Utilisation de la mutation consistant en la présence d'adénine, A, au lieu de thymine, T,
a. dans la position 1109 de la séquence nucléotidique du gène *nor* de la tomate SEQ ID NO: 1 ou
b. dans la position correspondant à la position 1109 de SEQ ID NO: 1 dans des séquences identiques à 80 % au moins à SEQ ID NO: 1,
en tant que marqueur pour identifier les plants de tomate possédant le génotype AA, dont les fruits, qui présentent des caractéristiques de longue durée de vie, peuvent être conservés pendant une période d'au moins 3 mois sans qu'il soit besoin de les réfrigérer.

2. L'utilisation selon la revendication 1, dans laquelle est en outre sélectionné le plant de tomate identifié possédant le génotype AA dans la position 1109, conformément à la description de la revendication 1 a ou 1 b.

3. L'utilisation selon la revendication 1, dans laquelle est en outre sélectionnée au moins une semence du plant de tomate identifié possédant le génotype AA dans la position 1109, conformément à la description de la revendication 1 a ou 1 b.

4. L'utilisation selon n'importe laquelle des revendications 1 à 3, dans laquelle le plant de tomate est classé taxonomiquement dans l'espèce *Solanum lycopersicum.*


### SEQUENCE LISTING

<110> Semillas Fitó, S.A.
<120> USE OF A MUTATION AS A MARKER FOR IDENTIFYING TOMATO PLANTS WHOSE FRUITS HAVE LONG-LIFE CHARACTERISTICS
<130> EP2096.3
<140> EP2255006
   <141> 2010-09-06
<150> PCT/EP2009/052912
   <151> 2009-03-12
<160> 11
<170> PatentIn version 3.5
<210> 1
   <211> 2885
   <212> DNA
   <213> Solanum lycopersicum
<400> 1
   ctaaattcct tcttgtttat cattttctct cttcccaaaa aaaaatccca aaatttaatc 60
   ataatacaat tcgaatttat caacctcgta ctacgtacat atttttgttg gtacgtaaaa 120
   tactgaattc aggtcaactc aaacatcgta aattgtgatt tctttatgga aagtacggat 180
   tcatcaaccg ggacacgtca tcagcctcaa ctcccaccgg ggtttcgatt ccacccgacg 240
   gacgaagaac tcatcgtcca ctacctcaaa aaacgagtcg ccggcgctcc gattccggtg 300
   gatattattg gtgaaattga tctttataag tttgatccat gggaactccc tgctactatt 360
   ttcaccacta tactatattt tcttgcccta taacttatat ataggggaaa aagatcggag 420
   tcagcgatga acaattattg tgtctaaatt aaattttaaa tatgcaatag attggtgacg 480
   aatttcgttg ctaattaatt ttttagtgat aaattaatat ttttcccctt tttaatcttc 540
   atgtttttta tcacaaagtt ttctatgacc aacttataaa gatttgaact cgatcaattt 600
   tttttttaga atgaatgaac ttatgttata tatagtgata ttttaaatgc ttttttatat 660
   tttcaaaaga tatccacgat aacgtgtaaa aagtgaattt gcaaaaaaaa aatgtagtac 720
   cttttattta attttattgt agataattta gattttaatt ttgaatttgt ttaatttaaa 780
   ttctgaatcg tataatattt atttaatttc tattttttga gttttttttt ggagggtgct 840
   taaaaagtag tattcacaaa tataaagtag tggacaaaca taaagtagtg gacccataat 900
   ttattttttt aaaaattata ttaaaactat ttgttaagtt taaattctga attatcttct 960
   tatcatgtgt ttaacgcagc taaggcaata ttcggagagc aagaatggtt cttttttagt 1020
   ccaagagata gaaaatatcc taacggggcg aggccaaatc gggctgcaac atcgggttat 1080
   tggaaggcta ccggaaccga caagccggtt tttacttccg gtggaacaca aaaggttggg 1140
   gtaaaaaagg cgctcgtttt ttacggcggt aaaccaccaa aaggggtaaa aactaattgg 1200
   atcatgcatg aatacagagt tgtagaaaat aaaacaaata acaagccact tggttgtgat 1260
   aatattgttg ccaacaaaaa aggatctttg agggtaagtc ctaaattttg catcgaaact 1320
   aatttctcta tcgtatcaga tagggataag atatacgtat actctaatct ccttgaacca 1380
   cacaagtact atactagata tgttgttgtt gtagatgact tgattcaact ttcaaatttt 1440
   tgatgaaaat gtttaagtta tatataccat atatatatag gcgtagctaa aaatttcgat 1500
   aaggggtttt aaatctgaaa aaatggatat acgaaatagc cgaaagaggt tcgacataga 1560
   ttattttaac catataaaaa taatacaatt ttcatatata tatacgccgt ggttaatatg 1620
   aggaatattt tatactatta atgtacttta accaggggcg gctctagagt tgatgaaccc 1680
   tctcagcgaa aatttacgtt gtatatttaa ggtacctttt aataattttt gtatttatat 1740
   attaattttg aacctcttga atataagatt agacgttgac ttagtggttt caggggttca 1800
   aatcactatt ctttttttcc taaccccctt aatgaaaatc ctgaatcggc cactaacttt 1860
   aactggttat agaaggttaa tcttactaga aaaaagcatg aaattctaac cgacaaagat 1920
   gtagtcgccc agttagataa gacgtttaaa ttgggcggat agagttactt tatttttcac 1980
   tgtcatatgt tactatatat tgacacttca cttaaagagt tatcatatcg atatttttac 2040
   tattagtgta cataacacaa actcgaataa attcaatgtt tcattagcta gttaattagt 2100
   ctaacttttt taaaaaaaaa tatttttctt actccacact attttatttt atttttttgc 2160
   agctagatga ttgggtttta tgtcgaattt acaagaagaa taacacacaa aggtccatag 2220
   atgatttgca tgatatgttg ggatcgatac cacaaaatgt accaaattca atattacaag 2280
   gaataaagcc ttcaaactat ggtacaatat tgctcgaaaa tgaatcgaat atgtacgatg 2340
   gaattatgaa taacacgaac gatattatca acaataataa tagatccatt ccacaaatat 2400
   cgtcaaagag aacgatgcat ggaggtttgt attggaataa cgacgaagca acaacaacaa 2460
   caacaactat tgataggaac cattctccaa atacaaaaag gttccttgtt gagaacaacg 2520
   aggacgatgg acttaacatg aataatattt cgcgaattac aaatcatgaa caaagtagct 2580
   ccattgccaa tttcctgagc cagtttcctc aaaatccttc gattcaacaa caacaacaac 2640
   aacaagaaga agtattggga tctcttaatg atggggtcgt ctttcgacaa ccttataatc 2700
   aagttactgg catgaattgg tactcttaaa gatataaaaa ggcaaaaaat agttagccct 2760
   gtaaaatcaa tcgatcaatc aatcatagat atattatata tggatttcgt tatattttac 2820
   ttttagttag aattaatata tagaatatct tctatctcac attaacaaat aagaacattt 2880
   ataac 2885
<210> 2
   <211> 1423
   <212> DNA
   <213> Solanum lycopersicum
<400> 2
   ctaaattcct tcttgtttat cattttctct cttcccaaaa aaaaaatccc aaaatttaat      60
   cataatacaa ttcgaattta tcaacctcgt actacgtaca tatttttgtt ggtacgtaaa     120
   atactgaatt caggtcaact caaacatcgt aaattgtgat ttctttatgg aaagtacgga     180
   ttcatcaacc gggacacgtc atcagcctca actcccaccg gggtttcgat tccacccgac     240
   ggacgaagaa ctcatcgtcc actacctcaa aaaacgagtc gccggcgctc cgattccggt     300
   ggatattatt ggtgaaattg atctttataa gtttgatcca tgggaactcc ctgctaaggc     360
   aatattcgga gagcaagaat ggttcttttt tagtccaaga gatagaaaat atcctaacgg     420
   ggcgaggcca aatcgggctg caacatcggg ttattggaag gctaccggaa ccgacaagcc     480
   ggtttttact tccggtggaa cacaaaaggt tggggtaaaa aaggcgctcg ttttttacgg     540
   cggtaaacca ccaaaagggg taaaaactaa ttggatcatg catgaataca gagttgtaga     600
   aaataaaaca aataacaagc cacttggttg tgataatatt gttgccaaca aaaaaggatc     660
   tttgaggcta gatgattggg ttttatgtcg aatttacaag aagaataaca cacaaaggtc     720
   catagatgat ttgcatgata tgttgggatc gataccacaa aatgtaccaa attcaatatt     780
   acaaggaata aagccttcaa actatggtac aatattgctc gaaaatgaat cgaatatgta     840
   cgatggaatt atgaataaca cgaacgatat tatcaacaat aataatagat ccattccaca     900
   aatatcgtca aagagaacga tgcatggagg tttgtattgg aataacgacg aagcaacaac     960
   aacaacaaca actattgata ggaaccattc tccaaataca aaaaggttcc ttgttgagaa    1020
   caacgaggac gatggactta acatgaataa tatttcgcga attacaaatc atgaacaaag    1080
   tagctccatt gccaatttcc tgagccagtt tcctcaaaat ccttcgattc aacaacaaca    1140
   acaacaacaa gaagaagtat tgggatctct taatgatggg gtcgtctttc gacaacctta    1200
   taatcaagtt actggcatga attggtactc ttaaagatat aaaaaggcaa aaaatagtta    1260
   gccctgtaaa atcaatcgat caatcaatca tagatatatt atatatggat ttcgttatat    1320
   tttactttta gttagaatta atatatagaa tatcttctat ctcacattaa caaataagaa    1380
   catttataac aaaaaaaaaa aaaaaaaaaa aaaaaaaaaa aaa                      1423
<210> 3
   <211> 22
   <212> DNA
   <213> Solanum lycopersicum
<400> 3
   tcattttctc tcttcccaaa aa 22
<210> 4
   <211> 22
   <212> DNA
   <213> Solanum lycopersicum
<400> 4
   catgaagatt aaaaagggga aa 22
<210> 5
   <211> 23
   <212> DNA
   <213> Solanum lycopersicum
<400> 5
   tccacgataa cgtgtaaaaa gtg 23
<210> 6
   <211> 25
   <212> DNA
   <213> Solanum lycopersicum
<400> 6
   ttttcatcaa aaatttgaaa gttga 25
<210> 7
   <211> 20
   <212> DNA
   <213> Solanum lycopersicum
<400> 7
   ttcggagagc aagaatggtt 20
<210> 8
   <211> 22
   <212> DNA
   <213> Solanum lycopersicum
<400> 8
   caaccaagtg gcttgttatt tg 22
<210> 9
   <211> 25
   <212> DNA
   <213> Solanum lycopersicum
<400> 9
   aactcgaata aattcaatgt ttcat 25
<210> 10
   <211> 22
   <212> DNA
   <213> Solanum lycopersicum
<400> 10
   tcgattgatt ttacagggct aa 22
<210> 11
   <211> 20
   <212> DNA
   <213> Solanum lycopersicum
<400> 11 20
   taccggaacc gacaagccgg 20